# EUROPEAN PATENT SPECIFICATION

(11) **EP 2 688 532 B1**
(45) Date of publication and mention of the grant of the patent: **16.09.2020**
(21) Application number: 12713299.1
(22) Date of filing: 20.03.2012
(51) Int. Cl.: A61F 9/008, A61F 9/009

(54) **APPARATUS AND METHOD FOR CONTROL OF REFRACTIVE INDEX CHANGES IN A MATERIAL**
VORRICHTUNG UND VERFAHREN ZUR KONTROLLE VON BRECHUNGSINDEXVERÄNDERUNGEN BEI EINEM MATERIAL
APPAREIL ET PROCÉDÉ POUR LA COMMANDE DE VARIATIONS D'INDICE DE RÉFRACTION DANS UNE MATIÈRE

(30) Priority: 24.03.2011 US 201161467263 P; 19.03.2012 US 201213423919
(43) Date of publication of application: 29.01.2014
(73) Proprietor: Technolas Perfect Vision GmbH, 80992 München (DE)
(72) Inventor: KANDULLA, Jochen, 80805 Muenchen (DE)
(74) Representative: Patent- und Rechtsanwälte Ullrich & Naumann
(86) International application number: PCT/IB2012/000549
(87) International publication number: WO 2012/127301

(56) References cited:
- EP-A2- 0 608 052
- WO-A1-2006/074898
- WO-A1-2012/031277
- US-A1- 2009 069 794

## Description

### FIELD OF THE INVENTION

The present invention pertains generally to systems and methods for performing ophthalmic surgery. More particularly, the present invention pertains to systems and methods for stabilizing an eye during ophthalmic surgery. The present invention is particularly, but not exclusively, useful as a system and method that stabilizes the eye with a contact element while causing minimal changes in refractive properties of the eye during ophthalmic surgery.

### BACKGROUND OF THE INVENTION

Surgical lasers are now commonly used in a variety of ophthalmic surgical procedures, including the treatment of ocular diseases and the correction of optical deficiencies. In these procedures, the surgical laser is often chosen as the tool of choice because of the ability of the laser to be accurately focused with great precision. In addition, the ability of the laser to be guided to designated locations within the eye, with precision and reliability, has enabled ophthalmic procedures to be performed throughout the eye.

Anatomical characteristics of the eye, however, can undermine the effectiveness of any laser procedure. In particular, this is so for ophthalmic laser surgery that is to be performed on tissue behind (i.e. posterior) the cornea. Specifically, the beam of a laser can be significantly degraded by wrinkles that may be induced predominantly on the posterior surface of the cornea of an eye, when the eye is being stabilized by a contact element. The effect of these wrinkles becomes most acute when the laser beam is used for procedures on tissues in the deeper regions of the eye beyond the cornea, such as the lens or the retina.

Typically, when an eye stabilizing device is used, it is placed against the anterior surface of the eye and is pressed in a posterior direction.

Such eye stabilizing devices are e.g. known from documents EP0608052, WO2006/074898, US2009/069794 and WO2012/031277 As a consequence, tissue in the eye may be squeezed in a manner that will cause wrinkles to be created primarily on the posterior surface of the cornea of the eye. These wrinkles can then cause an undesirable refraction, dispersion and degradation of the laser beam, as well as other adverse optical effects, as it passes through the cornea. An additional drawback caused by dispersion of the laser beam is the possibility of unintentionally damaging non-targeted tissue.

In light of the above, it is an object of the present invention to stabilize the eye for a laser surgical procedure with a contact element that avoids changing the refractive properties of the eye. Another object of the present invention is to properly position a contact element to minimize the distortion and degradation of a laser beam as it travels through the cornea to perform an ophthalmic procedure on tissue in the eye, particularly beyond the cornea. Yet another object of the present invention is to provide a device and method for stabilizing the eye during an ophthalmic procedure that is easy to use, is relatively simple to manufacture, and is comparatively cost effective.

### SUMMARY OF THE INVENTION

In accordance with the present invention, a system and method are provided for stabilizing an eye which require physically restraining movements of an eye in orthogonal x-y-z directions. The essential purpose here is to stabilize the eye, or some other transparent object made of a resilient material, while preventing any distortion of the eye (object) that will substantially change its refraction or refractive properties. For the present invention, this is done by juxtaposing the contact surface of a contact element against a selected surface of the eye (object) and establishing an operational location for the contact element relative to the eye. More specifically, with the contact element in its operational location there will be minimal, if any, contact pressure on the eye and, thus, unwanted distortions of the eye are effectively obviated.

Structurally, in order to establish a proper juxtaposition of the contact element, the contact surface is shaped with a substantially matching (i.e. mating) correspondence to the selected surface of the eye (object). For example, in a preferred embodiment of the present invention, the contact surface will be substantially concave, and the selected surface (e.g. the anterior surface of the cornea of the eye) will be substantially convex.

In addition to the contact element, the system includes a detector for monitoring an interaction between the contact element and the object. As intended for the present invention, one purpose of the detector is to establish and maintain an operational location for the contact element that will oppose movements of the eye (object). Another purpose of the detector is to generate a position signal that indicates an interaction between the contact element and the object, and that can be used by the system to ensure proper positioning of the contact element onto the eye. For the present invention, the detector may be either a pressure sensor, or an imaging unit.

In an embodiment of the system wherein the detector is a pressure sensor, the detector can be of any type well known in the pertinent art. Preferably, it will be mounted directly on the contact element. The operational location of the contact element can then be established whenever the contact element is pressed against the eye and a pressure reading, or position indicator, from the detector attains a predetermined value. As will be appreciated by a skilled artisan, this predetermined value will typically be based on various characteristic factors of the eye (object), such as surface topography, shape and type of material.

For an embodiment of the system wherein the detector is an imaging unit, the imaging unit will typically include a light source and a detector. For example, the present invention envisions OCT or Scheimpflug imaging. In any event, the light source will be used for directing an imaging light beam to both the contact element and to the eye (object). The imaging unit includes a receiving unit that will then receive light that is reflected from the contact element and from the object, and it will use this light to image the interaction between the contact element and the eye (object). Based on images of this interaction, the operational location of the contact element is established as being either: 1) when the eye (object) attains a predetermined shape after placement of the contact element (e.g. when a smooth posterior corneal surface is achieved); or 2) when the contact element makes initial contact with the eye (object). In both cases, the image can be used to determine when the distance between the contact element and the eye is equal to zero. For this embodiment, the contact element is preferably made of optical grade glass or a clear plastic material.

It will be appreciated by the skilled artisan that the present invention lends itself to feedback control during the placement of the contact element. When feedback control is used, a computer and a controller are provided to cooperatively establish the contact element in its proper location on the eye. To do this, the detector produces an image or some other indication (e.g. pressure reading) of the interaction between the contact element and the eye (object). This data is then communicated to the computer. Upon receipt of this data, the computer compares the data with a reference input. Specifically, the reference input will be the predetermined pressure value when a pressure sensor is used as the detector, and it will be imaging data (i.e. images) when an imaging unit is used as the detector. If the computer calculates a deviation when comparing the reference input with the position signal, an error signal is generated. When an error signal is generated, the controller will move the placing device to position the contact element at its operational location, which minimizes the deviation to establish the error signal as a null.

As an added feature of the present invention, a liquid can be deposited on the selected surface of the eye (object) prior to a juxtaposition of the contact element with the selected surface. Specifically, this can be done to buffer the interaction between the contact element and the object and further to equalize the pressure exerted by the contact element on the eye. The invention is defined in the appended claims.

### BRIEF DESCRIPTION OF THE DRAWINGS

Fig. 1 is a schematic presentation of a system for the present invention, wherein a contact element has been juxtaposed against the eye of a patient;
Fig. 2 is a cross sectional view of the contact element of the present invention in position relative to an eye of a patient as seen along line 2-2 in Fig. 1;
Fig. 3 is a cross sectional view of the contact element as shown in Fig. 2 when the contact element exerts excessive pressure on an eye causing undesirable changes to the refractive properties of the cornea;
Fig. 4 is a cross-sectional view of the contact element as shown in Fig. 2 with an imaging unit being used for an operational placement of the contact element against the eye; and
Fig. 5 is a cross-sectional view of the contact element as shown in Fig. 2 with a pressure sensor being used for an operational placement of the contact element against the eye.

### DESCRIPTION OF THE PREFERRED EMBODIMENTS

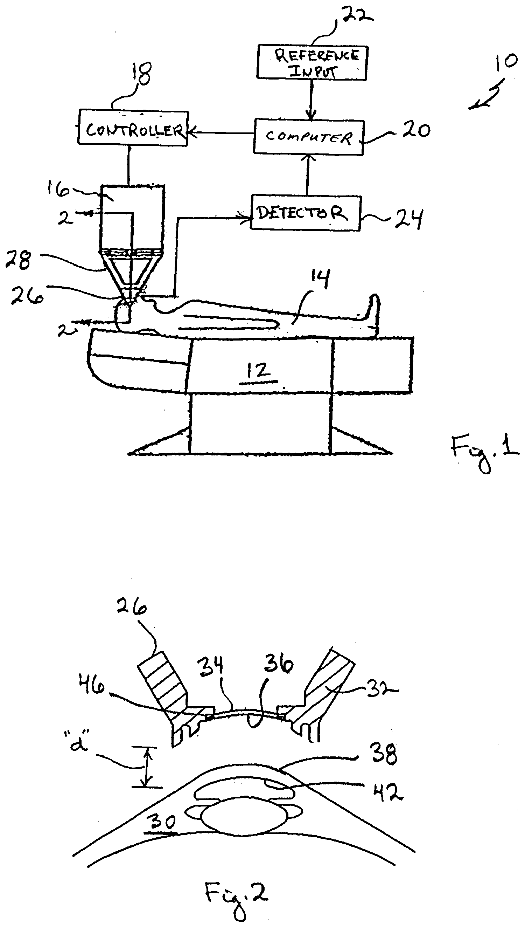

Referring initially to Fig. 1, a system for minimizing refractive index change in an eye (work piece) during an ophthalmic laser surgical procedure (alteration of the work piece) is shown and is generally designated 10. As shown, the system 10 includes a table (chair) 12 for supporting a patient 14 during an ophthalmic surgical laser procedure. The system 10 also includes a laser unit 16 for performing the surgical laser procedure. Further, system 10 includes a controller 18 for operating the laser unit 16, and it includes a computer 20 that provides instructions for an operation of the controller 18.

Fig. 1 also indicates that the computer 20 functions in response to a reference input 22, and that the computer 20 also receives input from a detector 24. More specifically, the detector 24 provides information to the computer 20 that pertains to the interactive relationship between a contact element 26 and the patient 14. In particular, this interactive relationship is monitored as the contact element 26 is moved by a placement device 28 (i.e. a device for placing) into contact with an eye 30 of the patient 14. The purpose here is to establish an operational relationship between the contact element 26 and the eye 30 that will stabilize the eye 30 during an ophthalmic laser procedure, without causing unwanted distortions of the eye 30.

The structural details of the contact element 26 will perhaps be best appreciated with reference to Fig. 2. There it will be seen that the contact element 26 includes a base 32, with a contact lens 34 that is mounted on the base 32. In detail, the contact lens 34 will typically have a contact surface 36 that substantially conforms to the shape of the anterior surface 38 of the eye 30. It is to be appreciated that this conformity (i.e. correspondence) will differ from patient to patient and, therefore, it may be desirable, but not necessarily mandatory, to customize the contact element 26 for a particular patient 14. Further, in order to be operationally compatible with the laser unit 16, it is envisioned that the contact lens 34 of the contact element 26 will preferably be made of either an optical grade glass of a clear medical grade plastic.

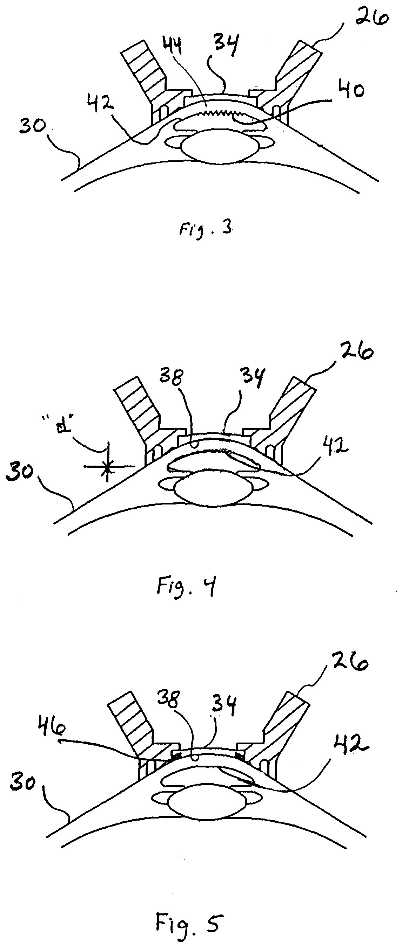

In an operation of the system 10, the objective is to prevent a condition such as is shown in Fig. 3, wherein wrinkles 40 are formed on the posterior surface 42 of the cornea 44. As indicated earlier, the avoidance of wrinkles 40 helps ensure the maximum operational capability of the laser unit 16. For the present invention, this is accomplished by monitoring the interaction between the contact element 26 and the anterior surface 38 of the eye 30, as the contact element 26 is being placed (juxtaposed) onto the eye 30.

Operationally, the system 10 monitors a distance "d" that is measured between the contact element 26 and the anterior surface 38 of the eye 30 (see Fig. 2). For purposes of the present invention, because the contact surface 36 of the contact lens 34 is shaped to substantially conform to the anterior surface 38 of the eye 30, the distance "d" will be substantially the same at every point on the anterior surface 38. In the eventuality that there may be detectable differences in the distance "d" between the contact lens 34 and eye 30, as it is measured between the contact surface 36 and the anterior surface 38, a fluid film (not shown) can be employed between the contact surface 36 and the anterior surface 38 to obviate the differences. In any event, the detector 24 is used to measure the distance "d", and to then provide this information to the computer 20. With information about the distance "d", the computer 20 compares this information with the reference input 22. Based on this comparison, the computer 20 defines an error signal that is dependent on the distance "d". Using well known closed loop feedback control techniques, the computer 20 then directs the controller 18 to move the laser unit 16, and the placement device 28, for placement of the contact element 26 into its operational location. For purposes of the present invention, the operational location of the contact element 26 is established when the contact surface 36 of the contact lens 34 is juxtaposed with the anterior surface 38 of the eye 30 (i.e. d=0), and the condition of the posterior surface 42 of the eye 30 shown in Fig. 3 is avoided (i.e. there are no wrinkles 40, or other structural distortions of the eye 30). In accordance with the present invention, this can be accomplished in either of two ways. For one, the detector 24 can be used as an inquiry unit. For another, the detector 24 can be used to detect pressures.

With reference to Fig. 4, and with cross reference back to Fig. 2, an embodiment for the system 10 is indicated wherein the detector 24 is an imaging unit. More specifically, for purposes of the present invention, an imaging system for use as the detector 24 can be of any type well known in the pertinent art, such as devices that employ techniques of Optical Coherence Tomography (OCT), Scheimpflug, two-photon imaging, wavefront analysis and non-optical techniques such as acoustical imaging. Regardless of type, however, the detector 24 is used to operationally observe the distance "d" (e.g. as shown in Fig. 2) and indicate when the distance "d" equals zero (e.g. when there is contact between the contact element 26 and the eye 30 as shown in Fig. 4). In detail, when d=0, the embodiment of system 10 that includes an "imaging" type detector 24 can react and indicate achievement of an operational location for the contact element 26 in either of two circumstances. For one, the operational location can be established for contact element 26 by reference input 22 when an image created by the detector 24 indicates that "d" is actually zero. For another, again based on a reference input 22, the operational location can be established for contact element 26 when an image indicates there has been a predetermined change in the shape of the cornea 44 of the eye 30.

With reference to Fig. 5, and with cross reference back to Fig. 2, an embodiment for the system 10 is indicated wherein the detector 24 is a "pressure activated" type detector 24. For this embodiment, a pressure sensor 46 is employed. Preferably, the pressure sensor 46 will be of a type well known in the pertinent art, and it will be mounted on the contact element 26 for contact with the anterior surface 38 of the cornea 44. In this case, the operational location for contact element 26 is established when the pressure sensor 46 indicates that the predetermined value for pressure of the contact element 26 against the anterior surface 38 of the cornea 44 has been attained. As implied above, the detector 24 can also respond as a position indicator when the pressure sensor 24 reacts with a movement to the interaction of the contact lens 34 with the eye 30.

It will be appreciated by the skilled artisan that a buffering fluid can be positioned on the anterior surface 38 of the eye 30 to distribute the interaction of the contact element 26 with the eye 30. This fluid (not shown) can be used for either embodiment of the present invention.

While the particular Apparatus and Method for Control of Refractive Index Changes in a Material as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system for stabilizing a resilient object to avoid operationally changing refractive properties of the object, the system comprising:
a contact element formed with a contact surface, wherein the contact surface is shaped with a substantially matching correspondence to a selected surface on the resilient object;
a device for placing the contact element onto the selected surface of the object, wherein the contact element is placed in an operational location when the contact surface of the contact element is juxtaposed with the selected surface of the object, and wherein any movement of the object is opposed by the contact element to stabilize the object while the contact element is in its operational location;
a detector for generating a position signal indicative of an interaction between the contact element and the object, wherein the position signal is used with the device to establish and maintain the contact element in its operational location to oppose movement of the object, **characterised in that** the system further comprises
a liquid, wherein the system is adapted to deposit the liquid on the selected surface of the object to buffer the interaction between the contact element and the object.

2. A system as recited in claim 1 further comprising:
a computer connected to the detector for receiving the position signal and for comparing the position signal with a reference input to generate an error signal; and
a controller for receiving the error signal from the computer and for moving the placing device to establish the error signal as a null.

3. A system as recited in claim 2 wherein the detector is a pressure sensor for indicating a pressure reading and the operational location of the contact element is established when the pressure reading of the sensor attains a predetermined value, and wherein the predetermined value is the reference input.

4. A system as recited in claim 3 wherein the predetermined value is based on properties of the object including surface topography, shape and type of material.

5. A system as recited in claim 2 wherein the detector is an imaging unit, and the imaging unit comprises:
a light source for directing an imaging beam to the contact element and to the object; and
a receiver unit for receiving light from the contact element and from the object to produce an image of the interaction between the contact element and the object, wherein the image determines a distance "d" between the contact element and the anterior surface of the eye, and wherein the reference input is established where the distance "d" is equal to zero.

6. A system as recited in claim 5 wherein the operational location of the contact element is established when the object attains a predetermined shape.

7. A system as recited in claim 1 wherein the contact surface is substantially concave and the selected surface is substantially convex or
wherein the object is an eye of a patient and the selected surface is an anterior surface of the eye or
wherein the contact element is made of a material selected from a group consisting of glass and a clear transparent plastic.

8. A method for stabilizing a resilient object made of a transparent material, to avoid changing refractive properties of the object, the method comprising the steps of:
providing a contact element formed with a contact surface, wherein the contact surface is shaped with a substantially matching correspondence to a selected surface on the resilient object;
juxtaposing the contact surface of the contact element with the selected surface on the object to establish an operational location for the contact element wherein any movement of the object, while the contact element is in its operational location, is opposed by the contact element to stabilize the object;
using a detector to generate a position signal indicative of an interaction between the contact element and the object, wherein the position signal is used to establish and maintain the contact element in its operational location to oppose movement of the object; and
using a liquid, wherein the liquid is deposited on the selected surface of the object to buffer the interaction between the contact element and the object.

9. A method as recited in claim 8 further comprising the steps of:
connecting a computer to the detector to receive the position signal;
using the computer to compare the position signal with a reference input to generate an error signal;
providing a controller to receive the error signal from the computer; and
moving the contact element with the controller to establish the error signal as a null.

10. A method as recited in claim 9 further comprising the steps of:
mounting the detector on the contact element, wherein the detector is a pressure sensor; and
verifying establishment of the operational location in the moving step by an indication of a predetermined value for a pressure reading obtained from the pressure sensor,
wherein preferably the reference input is the predetermined value for the pressure reading.

11. A method as recited in claim 8 further comprising the steps of:
directing an imaging beam toward the contact element and the object;
receiving reflections from the contact element, and from the object, to create an image of the interaction therebetween; and
using the image to determine a distance "d" between the contact element and the anterior surface of the object, wherein the distance "d" equals the error signal and the contact element is in its operational location when the distance "d" is equal to zero.

## Patentansprüche

1. System zum Stabilisieren eines elastischen Objekts, um sich betriebsbedingt ändernde Brechungseigenschaften des Objekts zu vermeiden, wobei das System umfasst:
ein Kontaktelement, das mit einer Kontaktfläche gebildet ist, wobei die Kontaktfläche mit einer im Wesentlichen übereinstimmenden Entsprechung zu einer ausgewählten Fläche an dem elastischen Objekt geformt ist;
eine Vorrichtung zum Platzieren des Kontaktelements auf der ausgewählten Fläche des Objekts, wobei das Kontaktelement in einer Betriebsposition platziert wird, wenn die Kontaktfläche des Kontaktelements mit der ausgewählten Fläche des Objekts aneinandergereiht wird, und wobei jeder Bewegung des Objekts durch das Kontaktelement entgegengewirkt wird, um das Objekt zu stabilisieren, während sich das Kontaktelement in seiner Betriebsposition befindet;
einen Detektor zum Erzeugen eines Positionssignals, das eine Wechselwirkung zwischen dem Kontaktelement und dem Objekt anzeigt, wobei das Positionssignal mit der Vorrichtung verwendet wird, um das Kontaktelement in seiner Betriebsposition einzurichten und aufrechtzuerhalten, um einer Bewegung des Objekts entgegenzuwirken, **dadurch gekennzeichnet, dass** das System ferner eine Flüssigkeit umfasst, wobei das System angepasst ist, die Flüssigkeit auf die ausgewählte Fläche des Objekts abzuscheiden, um die Wechselwirkung zwischen dem Kontaktelement und dem Objekt zu puffern.

2. System nach Anspruch 1, ferner umfassend:
einen Computer, der mit dem Detektor verbunden ist, zum Empfangen des Positionssignals und zum Vergleichen des Positionssignals mit einem Referenzeingang, um ein Fehlersignal zu erzeugen; und
eine Steuerung zum Empfangen des Fehlersignals von dem Computer und zum Bewegen der Platzierungsvorrichtung, um das Fehlersignal als eine Null einzurichten.

3. System nach Anspruch 2, wobei der Detektor ein Drucksensor zum Anzeigen einer Druckmessung ist und die Betriebsposition des Kontaktelements eingerichtet wird, wenn die Druckmessung des Sensors einen vorbestimmten Wert erreicht, und wobei der vorbestimmte Wert der Referenzeingang ist.

4. System nach Anspruch 3, wobei der vorbestimmte Wert auf Eigenschaften des Objekts einschließlich Flächentopographie, Form und Art des Materials basiert.

5. System nach Anspruch 2, wobei der Detektor eine Bildgebungseinheit ist und die Bildgebungseinheit umfasst:
eine Lichtquelle zum Richten eines Bildgebungsstrahls auf das Kontaktelement und das Objekt; und
eine Empfängereinheit zum Empfangen von Licht von dem Kontaktelement und von dem Objekt, um ein Bild der Wechselwirkung zwischen dem Kontaktelement und dem Objekt zu erzeugen, wobei das Bild einen Abstand "d" zwischen dem Kontaktelement und der vorderen Fläche des Auges bestimmt, und wobei die Referenzeingabe eingerichtet ist, wenn der Abstand "d" gleich Null ist.

6. System nach Anspruch 5, wobei die Betriebsposition des Kontaktelements eingerichtet ist, wenn das Objekt eine vorbestimmte Form erreicht.

7. System nach Anspruch 1, wobei die Kontaktfläche im Wesentlichen konkav ist und die ausgewählte Fläche im Wesentlichen konvex ist, oder
wobei das Objekt ein Auge eines Patienten ist und die ausgewählte Fläche eine vordere Fläche des Auges ist, oder
wobei das Kontaktelement aus einem Material hergestellt ist, das ausgewählt ist aus einer Gruppe bestehend aus Glas und einem klaren transparenten Kunststoff.

8. Verfahren zum Stabilisieren eines elastischen Objekts, das aus einem transparenten Material hergestellt ist, um sich ändernde Brechungseigenschaften des Objekts zu vermeiden, wobei das Verfahren die Schritte umfasst
Bereitstellen eines Kontaktelements, das mit einer Kontaktfläche gebildet ist, wobei die Kontaktfläche mit einer im Wesentlichen übereinstimmenden Entsprechung zu einer ausgewählten Fläche an dem elastischen Objekt geformt ist;
Aneinanderreihen der Kontaktfläche des Kontaktelements mit der ausgewählten Fläche an dem Objekt, um eine Betriebsposition für das Kontaktelement einzurichten, wobei jeder Bewegung des Objekts durch das Kontaktelement entgegengewirkt wird, während sich das Kontaktelement in seiner Betriebsposition befindet, um das Objekt zu stabilisieren;
Verwenden eines Detektors, um ein Positionssignal zu erzeugen, das eine Wechselwirkung zwischen dem Kontaktelement und dem Objekt anzeigt, wobei das Positionssignal verwendet wird, um das Kontaktelement in seiner Betriebsposition einzurichten und zu halten, um einer Bewegung des Objekts entgegenzuwirken; und
Verwenden einer Flüssigkeit, wobei die Flüssigkeit auf der ausgewählten Fläche des Objekts abgeschieden wird, um die Wechselwirkung zwischen dem Kontaktelement und dem Objekt zu puffern.

9. Verfahren nach Anspruch 8, ferner umfassend die Schritte:
Verbinden eines Computers mit dem Detektor, um das Positionssignal zu empfangen;
Verwenden des Computers, um das Positionssignal mit einem Referenzeingang zu vergleichen, um ein Fehlersignal zu erzeugen;
Bereitstellen einer Steuerung, um das Fehlersignal von dem Computer zu empfangen; und
Bewegen des Kontaktelements mit der Steuerung, um das Fehlersignal als eine Null einzurichten.

10. Verfahren nach Anspruch 9, ferner umfassend die Schritte:
Anbringen des Detektors an dem Kontaktelement, wobei der Detektor ein Drucksensor ist; und
Verifizieren der Einrichtung der Betriebsposition in dem Bewegungsschritt durch eine Anzeige eines vorbestimmten Werts für eine von dem Drucksensor erhaltene Druckmessung,
wobei der Referenzeingang bevorzugt der vorbestimmte Wert für die Druckmessung ist.

11. Verfahren nach Anspruch 8, ferner umfassend die Schritte:
Richten eines Bildgebungsstrahls auf das Kontaktelement und das Objekt;
Empfangen von Reflexionen von dem Kontaktelement und von dem Objekt, um ein Bild der Wechselwirkung dazwischen zu erzeugen; und
Verwenden des Bildes, um einen Abstand "d" zwischen dem Kontaktelement und der vorderen Fläche des Objekts zu bestimmen, wobei der Abstand "d" gleich dem Fehlersignal ist und das Kontaktelement sich in seiner Betriebsposition befindet, wenn der Abstand "d" gleich Null ist.

## Revendications

1. Système pour la stabilisation d'un objet résilient afin d'éviter le changement opérationnellement des propriétés de réfraction de l'objet, le système comprenant :
un élément de contact formé avec une surface de contact, dans lequel la surface de contact est formée avec une correspondance correspondant sensiblement à une surface sélectionnée sur l'objet résilient ;
un dispositif pour le placement de l'élément de contact sur la surface sélectionnée de l'objet, dans lequel l'élément de contact est placé dans un emplacement opérationnel lorsque la surface de contact de l'élément de contact est juxtaposée avec la surface sélectionnée de l'objet, et dans lequel un quelconque mouvement de l'objet est opposé par l'élément de contact pour stabiliser l'objet alors que l'élément de contact se trouve dans son emplacement opérationnel ;
un détecteur pour la génération d'un signal de position indicatif d'une interaction entre l'élément de contact et l'objet, dans lequel le signal de position est utilisé avec le dispositif pour établir et maintenir l'élément de contact dans son emplacement opérationnel pour s'opposer à un mouvement de l'objet, **caractérisé en ce que** le système comprend en outre un liquide, dans lequel le système est adapté pour déposer le liquide sur la surface sélectionnée de l'objet pour tamponner l'interaction entre l'élément de contact et l'objet.

2. Système selon la revendication 1, comprenant en outre :
un ordinateur connecté au détecteur pour la réception du signal de position et pour la comparaison du signal de position avec une entrée de référence pour générer un signal d'erreur ; et
un contrôleur pour la réception du signal d'erreur à partir de l'ordinateur et pour le déplacement du dispositif de placement afin d'établir le signal d'erreur comme nul.

3. Système selon la revendication 2, dans lequel le détecteur est un capteur de pression pour l'indication d'une lecture de pression et l'emplacement opérationnel de l'élément de contact est établi lorsque la lecture de pression du capteur atteint une valeur prédéterminée, et dans lequel la valeur prédéterminée est l'entrée de référence.

4. Système selon la revendication 3, dans lequel la valeur prédéterminée est sur base des propriétés de l'objet comprenant la topographie de surface, la forme et le type de matériau.

5. Système selon la revendication 2, dans lequel le détecteur est une unité d'imagerie, et l'unité d'imagerie comprend :
une source lumineuse pour la conduite d'un faisceau d'imagerie vers l'élément de contact et vers l'objet ; et
une unité de réception pour la réception de la lumière à partir de l'élément de contact et à partir de l'objet pour produire une image de l'interaction entre l'élément de contact et l'objet, dans laquelle l'image détermine une distance « d » entre l'élément de contact et la surface antérieure de l'œil, et dans lequel l'entrée de référence est établie là où la distance « d » est égale à zéro.

6. Système selon la revendication 5, dans lequel l'emplacement opérationnel de l'élément de contact est établi lorsque l'objet atteint une forme prédéterminée.

7. Système selon la revendication 1, dans lequel la surface de contact est sensiblement concave et la surface sélectionnée est sensiblement convexe ou
dans lequel l'objet est un œil d'un patient et la surface sélectionnée est une surface antérieure de l'œil ou
dans lequel l'élément de contact est fait d'un matériau choisi parmi un groupe constitué de verre et d'un plastique limpide transparent.

8. Procédé pour la stabilisation d'un objet résilient fait d'un matériau transparent, pour éviter le changement des propriétés de réfraction de l'objet, le procédé comprenant les étapes de :
la fourniture d'un élément de contact formé avec une surface de contact, dans laquelle la surface de contact est formée avec une correspondance correspondant sensiblement à une surface sélectionnée sur l'objet résilient ;
la juxtaposition de la surface de contact de l'élément de contact avec la surface sélectionnée sur l'objet pour établir un emplacement opérationnel pour l'élément de contact dans lequel un quelconque mouvement de l'objet, alors que l'élément de contact est dans son emplacement opérationnel, est opposé par l'élément de contact pour stabiliser l'objet ;
l'utilisation d'un détecteur pour générer un signal de position indicatif d'une interaction entre l'élément de contact et l'objet, dans lequel le signal de position est utilisé pour établir et maintenir l'élément de contact dans son emplacement opérationnel pour s'opposer au mouvement de l'objet ; et
l'utilisation d'un liquide, dans lequel le liquide est déposé sur la surface sélectionnée de l'objet pour tamponner l'interaction entre l'élément de contact et l'objet.

9. Procédé selon la revendication 8, comprenant en outre les étapes de :
la connexion d'un ordinateur au détecteur pour recevoir le signal de position ;
l'utilisation de l'ordinateur pour comparer le signal de position avec une entrée de référence pour générer un signal d'erreur ;
la fourniture d'un contrôleur pour recevoir le signal d'erreur à partir de l'ordinateur ; et
le déplacement de l'élément de contact avec le contrôleur pour établir le signal d'erreur comme nul.

10. Procédé selon la revendication 9, comprenant en outre les étapes de :
le montage du détecteur sur l'élément de contact, dans lequel le détecteur est un capteur de pression ; et
la vérification de l'établissement de l'emplacement opérationnel dans l'étape de déplacement par une indication d'une valeur prédéterminée pour une lecture de pression obtenue à partir du capteur de pression,
dans lequel de préférence l'entrée de référence est la valeur prédéterminée pour la lecture de pression.

11. Procédé selon la revendication 8, comprenant en outre les étapes de :
la conduite d'un faisceau d'imagerie vers l'élément de contact et l'objet ;
la réception de réflexions à partir de l'élément de contact, et à partir de l'objet, pour créer une image de l'interaction entre eux ; et
l'utilisation de l'image pour déterminer une distance « d » entre l'élément de contact et la surface antérieure de l'objet, dans lequel la distance « d » est égale au signal d'erreur et l'élément de contact est dans son emplacement opérationnel lorsque la distance « d » est égale à zéro.
